# EUROPEAN PATENT SPECIFICATION

(11) **EP 1 017 690 B1**
(45) Date of publication and mention of the grant of the patent: **05.12.2001**
(21) Application number: 98942342.1
(22) Date of filing: 01.09.1998
(51) Int. Cl.: C07D 311/92, C07D 407/04, C07D 409/04, G02B 5/23

(54) **NOVEL AROMATIC SUBSTITUTED NAPHTHOPYRANS**
AROMATISCHE SUBSTITUIERTE NAPHTOPYRANEN
NOUVEAUX NAPHOTPYRANS AROMATIQUES SUBSTITUES

(30) Priority: 19.09.1997 US 933556
(43) Date of publication of application: 12.07.2000
(73) Proprietor: PPG Industries Ohio, Inc., Cleveland, OH 44111 (US)
(72) Inventor: LIN, Jibing, St. Charles, IL 60175 (US)
(74) Representative: Sternagel, Fleischer, Godemeyer & Partner Patentanwälte
(86) International application number: US9818110
(87) International publication number: WO9915519

(56) References cited:
- WO-A-92/01959
- US-A- 5 658 501

## Description

The present invention relates to certain novel naphthopyran compounds. More particularly, this invention relates to novel photochromic aromatic substituted naphthopyran compounds and to compositions and articles containing such novel naphthopyran compounds. When exposed to electromagnetic radiation containing ultraviolet rays, such as the ultraviolet radiation in sunlight or the light of a mercury lamp, many photochromic compounds exhibit a reversible change in color. When the ultraviolet radiation is discontinued, such a photochromic compound will return to its original color or colorless state.

Various classes of photochromic compounds have been synthesized and suggested for use in applications in which a sunlight-induced reversible color change or darkening is desired. U.S. Patent 3,567,605 (Becker) describes a series of pyran derivatives, including certain benzopyrans and naphthopyrans. These compounds are described as derivatives of chromene and are reported to undergo a color change, e.g., from colorless to yellow-orange, on irradiation by ultraviolet light at temperatures below about -30°C. Irradiation of the compounds with visible light or upon raising the temperature to above about 0°C is reported to reverse the coloration to a colorless state.

U.S. Patent 5,066,818 describes various 3,3-diaryl-3H-naphtho[2,1-b]pyrans as having desirable photochromic properties, i.e., high colorability and acceptable fade, for ophthalmic and other applications. Also disclosed by way of comparative example in the '818 patent are the isomeric 2,2-diaryl-2H-naphtho[1,2-b]pyrans, which are reported to require unacceptably long periods of time to fade after activation.

U.S. Patent 3,627,690 describes photochromic 2,2-di-substituted-2H-naphtho[1,2-b]pyran compositions containing minor amounts of either a base or weak-to-moderate strength acid. The addition of either an acid or base to the naphthopyran composition is reported to increase the fade rate of the colored naphthopyrans, thereby making them useful in eye protection applications such as sunglasses. It is reported therein further that the fade rate of 2H-naphtho-[1,2-b]pyrans without the aforementioned additives ranges from several hours to many days to reach complete reversion.

U.S. Patent 4,818,096 discloses purple/blue coloring photochromic benzo- or naphthopyrane having at the position alpha to the oxygen of the pyran ring a phenyl group having a nitrogen containing substituent in the ortho or para positions. U.S. Patent 5,645,767 describes novel photochromic indeno-fused 2H-naphtho[1,2-b]pyran compounds, the 2,1-positions of the indeno group being fused to the f side of the naphthopyran. U.S. Patent 5,658,501 describes certain 2H-naphtho[1,2-b]pyran compounds having substituents at the 5 and 6 positions and possible substituents at the 7, 8, 9 and/or 10 positions, as well as substituents on the carbon atom adjacent to the oxygen of the pyran ring.

The following four patents disclose related photochromic 2H-naphtho[1,2-b]pyran compounds with certain substituents at the 2 position and certain aromatic substituents at the 5 position. U.S. Patent 4,826,977 discloses naphthopyrans having an adamantare group at the 2 position, a phenyl or substituted phenyl group or a 5- or 6-membered heteroaromatic group at the 5 position. U.S. Patent 4,931,221 discloses naphthopyrans having two cyclopropyl groups at the 2 position, and a phenyl or substituted phenyl group at the 5 position. U.S. Patent 4,980,089 describes naphthopyrans having a norcamphor group or a tricyelodecane group at the 2 position, and a furyl group, thienyl group or phenyl or substituted phenyl group at the 5 position. U.S. Patent 5,200,116 describes naphthopyrens having a cyclopropyl group along with a phenyl or substituted phenyl group, thienyl, benzothienyl group, or furyl or benzofuryl group at the 2 position and a phenyl or substituted phenyl group at the 5 position. The substituents of the 5 position phenyl groups disclosed in these four patents are C₁-C₄ alkyl, C₁-C₄ alkoxy, chloro or bromo.

U.S. Patent 5,458,814 discloses photochromic 2H-naphtho[1,2-b]pyran compounds having certain substituents at the number 5 and 6 carbon atoms of the naphtho portion of the naphthopyran, and at the 2-position of the pyran ring. These compounds have an acceptable fade rats in addition to a high activated intensity and a high coloration rate.

The present invention relates to novel 5-aromatic substituted 2H-naphtho[1,2-b]pyran compounds having certain substituents at the 2 position of the pyran ring. The arometic group at the 5-position of the naphthopyran is a substituted or unsubstituted phenyl or naphthyl group or other substituted or unsubstituted heteroaromatic group. Photochromic compounds of the present invention include compounds that exhibit acceptable photochromic performance properties, i.e., activated intensity, coloration rate and fade rate.

### DETAILED DESCRIPTION OF THE INVENTION

In recent years, photochromic plastic materials, particularly plastic materials for optical applications, have been the subject of considerable attention. In particular, photochromic ophthalmic plastic lenses have been investigated because of the weight advantage they offer, vis-à-vis, glass lenses. Moreover, photochromic transparencies for vehicles, such as cars and airplanes, have been of interest because of the potential safety features that such transparencies offer.

In accordance with the present invention, it has now been discovered that certain novel 5-aromatic substituted naphtho[1,2-b]pyrans having an activated yellow-orange color may be prepared. These compounds may be described as naphthopyrans having at the number 5 carbon atom of the naphthopyran a substituted or unsubstituted phenyl or naphthyl group or another substituted or unsubstituted heteroaromatic substituent, and certain substituents at the 2 position of the pyran ring. Certain substituents may also be present at the number 7, 8, 9 or 10 carbon atoms of the naphthopyran compound. The number 6 carbon atom of the naphthopyran compound has a hydrogen substituent.

The foregoing described naphthopyran compounds may be represented by the following graphic formula I in which the numbers 1 through 10 identify the ring atoms of the naphthopyran.

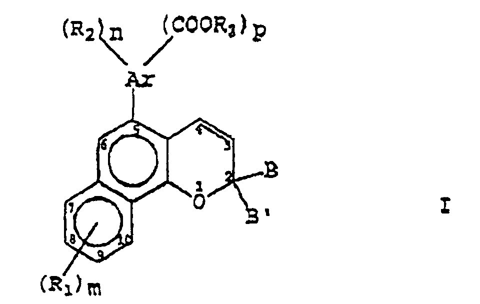

In graphic formula I, each R₁ may be C₁-C₆ alkyl, C₁-C₆ alkoxy, bromo, chloro or fluoro, and m is the integer 0, 1, 2 or 3. Preferably, each R₁ is C₁-C₄ alkyl, C₁-C₄ alkoxy, chloro or fluoro, and m is the integer 0, 1 or 2. Most preferably, each R₁ is C₁-C₃ alkyl or C₁-C₃ alkoxy, and m is the integer 0 or 1.

Ar in graphic formula I may be selected from the group consisting of:
(i) the aryl groups, phenyl and naphthyl; and
(ii) the heteroaromatic groups, furanyl, benzofuran-2-yl, benzofuran-3-yl, thienyl, benzothien-2-yl, benzothien-3-yl, dibenzofuran-4-yl, and dibenzothien-4-yl. Preferably, Ar is phenyl or thienyl.

Each R₂ in graphic formula I may be selected from the group consisting of aryl, i.e., phenyl and naphthyl, mono (C₁-C₆) alkoxyaryl, di(C₁-C₆)alkoxyaryl, mono(C₁-C₆)alkylaryl, di(C₁-C₆)alkylaryl, bromoaryl, chloroaryl, fluoroaryl, C₃-C₇ cycloalkylaryl, C₃-C₇ cycloalkyl, C₃-C₇ cycloalkyloxy, C₃-C₇ cycloalkyloxy(C₁-C₆)alkyl, C₃-C₇ cycloalkyloxy(C₁-C₆)alkoxy aryl(C₁-C₆)alkyl, aryl(C₁-C₆)alkoxy, aryloxy, aryloxy(C₁-C₆)alkyl, aryloxy(C₁-C₆)alkoxy, mono- and di(C₁-C₆)alkylaryl(C₁-C₆)alkyl, mono- and di(C₁-C₆)alkoxyaryl(C₁-C₆)alkyl, mono- and di(C₁-C₆)alkylaryl(C₁-C₆)alkoxy, mono- and di(C₁-C₆)alkoxyaryl(C₁-C₆)alkoxy, C₁-C₆ alkyl, C₁-C₆ bromoalkyl, C₁-C₆ chloroalkyl, C₁-C₆ fluoroalkyl, C₁-C₆ alkoxy, mono(C₁-C₆)alkoxy(C₁-C₄)alkyl, bromo, chloro and fluoro and n is the integer 0, 1, 2, or 3. Preferably, each R₂ is selected from the group consisting of aryl, aryloxy, aryl(C₁-C₃)alkyl, C₁-C₃ alkyl, C₁-C₃ chloroalkyl, C₁-C₃ fluoroalkyl, C₁-C₃ alkoxy, mono(C₁-C₃)alkoxy(C₁-C₃)alkyl, fluoro and chloro and n is the integer 0, 1 or 2. More preferably, each R₂ is selected from the group consisting of C₁-C₃ alkyl, C₁-C₃ alkoxy, aryl, fluoro and chloro and n is the integer 0 or 1.

R₃ in graphic formula I may be C₁-C₄ alkyl, phenyl, mono(C₁-C₄)alkyl substituted phenyl, mono(C₁-C₄)alkoxy substituted phenyl, phenyl(C₁-C₂)alkyl, mono(C₁-C₄)alkyl substituted phenyl(C₁-C₂)alkyl, mono(C₁-C₄)alkoxy substituted phenyl(C₁-C₂)alkyl, mono(C₁-C₄)alkoxy(C₂-C₃)alkyl, or C₁-C₄ haloalkyl, and p is the integer 0, 1 or 2, provided that the sum of n and p is less than or equal to 3 and when p is 2, n is 0. Preferably R₃ is C₁-C₃ alkyl and p is the integer 0 or 1. More preferably, the ester group containing R₃ is ortho to the atom of the Ar group linked to the number 5 carbon atom of the naphtho portion of the naphthopyran.

B and B' in graphic formula I may each be selected from the group consisting of:
(i) the unsubstituted, mono-, di- and trisubstituted aryl groups, phenyl and naphthyl;
(ii) the unsubstituted, mono- and di-substituted heteroaromatic groups pyridyl, furanyl, benzofuran-2-yl, benzofuran-3-yl, thienyl, benzothien-2-yl, benzothien-3-yl, dibenzofuran-4-yl, dibenzothien-4-yl, and carbazol-4-yl, each of said aryl and heteroaromatic substituents in parts (i) and (ii) being selected from the group consisting of hydroxy, aryl, mono(C₁-C₆)alkoxyaryl, di(C₁-C₆)alkoxyaryl, mono(C₁-C₆)alkylaryl, di(C₁-C₆)alkylaryl, bromoaryl, chloroaryl, fluoroaryl, C₃-C₇ cycloalkylaryl, C₃-C₇ cycloalkyl, C₃-C₇ cycloalkyloxy, C₃-C₇ cycloalkyloxy(C₁-C₆)alkyl, C₃-C₇ cycloalkyloxy(C₁-C₆)alkoxy, aryl(C₁-C₆)alkyl, aryl(C₁-C₆)alkoxy, aryloxy, aryloxy(C₁-C₆)alkyl, aryloxy(C₁-C₆)alkoxy, mono- and di(C₁-C₆)alkylaryl(C₁-C₆)alkyl, mono- and di(C₁-C₆)alkoxyaryl (C₁-C₆)alkyl, mono- and di(C₁-C₆)alkylaryl(C₁-C₆)alkoxy, mono- and di(C₁-C₆)alkoxyaryl(C₁-C₆)alkoxy, amino, mono(C₁-C₆)alkylamino, di(C₁-C₆)alkylamino, diarylamino, N-(C₁-C₆)alkylpiperazino, N-arylpiperazino, aziridino, indolino, piperidino, arylpiperidino, morpholino, thiomorpholino, tetrahydroquinolino, tetrahydroisoquinolino, pyrryl, C₁-C₆ alkyl, C₁-C₆ bromoalkyl, C₁-C₆ chloroalkyl, C₁-C₆ fluoroalkyl, C₁-C₆ alkoxy, mono(C₁-C₆)alkoxy(C₁-C₄)alkyl, acryloxy, methacryloxy, bromo, chloro and fluoro;
(iii) the groups represented by the following graphic formulae: 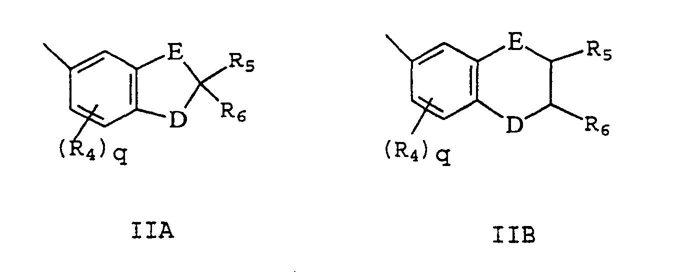 wherein E may-be carbon or oxygen and D may be oxygen or substituted nitrogen, provided that when D is substituted nitrogen, E is carbon, said nitrogen substituents being selected from the group consisting of hydrogen, C₁-C₆ alkyl and C₂-C₆ acyl; each R₄ is C₁-C₆ alkyl, C₁-C₆ alkoxy, hydroxy, bromo, chloro or fluoro; R₅ and R₆ are each hydrogen or C₁-C₆ alkyl; and q is the integer 0, 1 or 2;
(iv) C₁-C₆ alkyl, C₁-C₆ bromoalkyl, C₁-C₆ chloroalkyl, C₁-C₆ fluoroalkyl, C₁-C₆ alkoxy(C₁-C₄)alkyl; and
(v) the group represented by the following graphic formula: 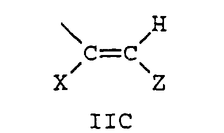
wherein X in graphic formula IIC may be hydrogen or C₁-C₄ alkyl and Z in graphic formula IIC may be selected from the unsubstituted, mono-, and di-substituted members of the group consisting of naphthyl, phenyl, furanyl and thienyl, each of said group substituents in this part (v) being C₁-C₄ alkyl, C₁-C₄ alkoxy, bromo, fluoro or chloro.

More preferably, B and B' are each selected from the group consisting of: (i) phenyl, mono-substituted phenyl and di-substituted phenyl, preferably substituted in the meta and/or para positions; (ii) the unsubstituted, mono- and di-substituted heteroaromatic groups furanyl, benzofuran-2-yl, thienyl, benzothien-2-yl, dibenzofuran-2-yl and dibenzothien-2-yl, each of said phenyl and heteroaromatic substituents in (i) and (ii) being selected from the group consisting of hydroxy, aryl, aryloxy, aryl(C₁-C₃)alkyl, amino, mono(C₁-C₃)alkylamino, di(C₁-C₃)alkylamino, N-(C₁-C₃)alkylpiperazino, indolino, piperidino, morpholino, pyrryl, C₁-C₃ alkyl, C₁-C₃ chloroalkyl, C_{C}-C-₃ fluoroalkyl, C₁-C₃ alkoxy, mono(C₁-C₃)alkoxy(C₁-C₃)alkyl, fluoro and chloro; (iii) the groups represented by the graphic formulae IIA and IIB, wherein E is carbon and D is oxygen, R₄ is C₁-C₃ alkyl or C₁-C₃ alkoxy, R₅ and R₆ are each hydrogen or C₁-C₄ alkyl; and q is the integer 0 or 1; (iv) C₁-C₄ alkyl; and (v) the group represented by the graphic formula IIC wherein X is hydrogen or methyl and Z is phenyl or mono-substituted phenyl, said phenyl substituent being selected from the group consisting of C₁-C₃ alkyl, C₁-C₃ alkoxy and fluoro.

Most preferably, B and B' are each selected from the group consisting of (i) phenyl, mono- and di-substituted phenyl; (ii) the unsubstituted, mono- and di-substituted heteroaromatic groups furanyl, benzofuran-2-yl, thienyl and benzothien-2-yl, each of said phenyl and heteroaromatic substituents in (i) and (ii) being selected from the group consisting of hydroxy, C₁-C₃ alkyl, C₁-C₃ alkoxy, aryl, indolino, fluoro and chloro; and (iii) the group represented by graphic formula IIA, wherein E is carbon and D is oxygen, R₄ is C₁-C₃ alkyl or C₁-C₃ alkoxy, R₅ and R₆ are each hydrogen or C₁-C₃ alkyl, and q is the integer 0 or 1.

Compounds represented by graphic formula I may be prepared by the following described Reactions A through D. Compounds represented by graphic formula V or VA are either purchased or prepared by Friedel-Crafts methods shown in Reaction A using an appropriately substituted or unsubstituted benzoyl chloride of graphic formula IV with a commercially available substituted or unsubstituted benzene compound of graphic formula III. See the publication Friedel-Crafts and Related Reactions, George A. Olah, Interscience Publishers, 1964, Vol. 3, Chapter XXXI (Aromatic Ketone Synthesis), and "Regioselective Friedel-Crafts Acylation of 1,2,3,4-Tetrahydroquinoline and Related Nitrogen Heterocycles: Effect on NH Protective Groups and Ring Size" by Ishihara, Yugi et al, J. Chem. Soc., Perkin Trans. 1, pages 3401 to 3406, 1992.

In Reaction A, the compounds represented by graphic formulae III and IV are dissolved in a solvent, such as carbon disulfide or methylene chloride, and reacted in the presence of a Lewis acid, such as aluminum chloride or tin tetrachloride, to form the corresponding substituted benzophenone represented by graphic formula V (or VA in Reaction B). R and R' represent possible substituents, as described hereinbefore with respect to graphic formula I.

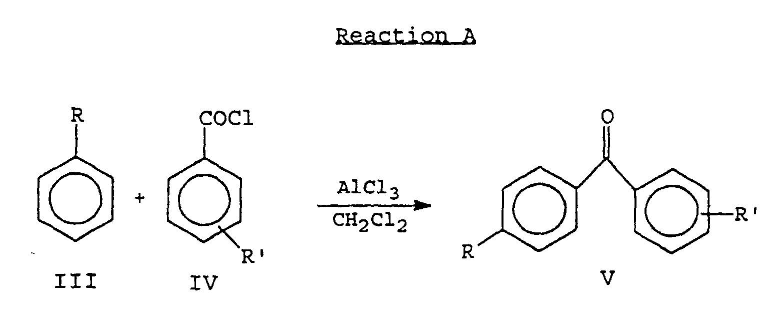

In Reaction B, the substituted or unsubstituted ketone represented by graphic formula VA, in which B and B' may represent groups other than substituted or unsubstituted phenyl, as shown in graphic formula V, is reacted with sodium acetylide in a suitable solvent, such as anhydrous tetrahydrofuran (THF), to form the corresponding propargyl alcohol represented by graphic formula VI. Propargyl alcohols having B or B' groups other than substituted and unsubstituted phenyl may be prepared from commercially available ketones or ketones prepared via reaction of an acyl halide with a substituted or unsubstituted benzene, naphthalene or heteroaromatic compound. Propargyl alcohols having a B or B' group represented by graphic formula IIC may be prepared by the methods described in U.S. Patent 5,274,132, column 2, lines 40 to 68.

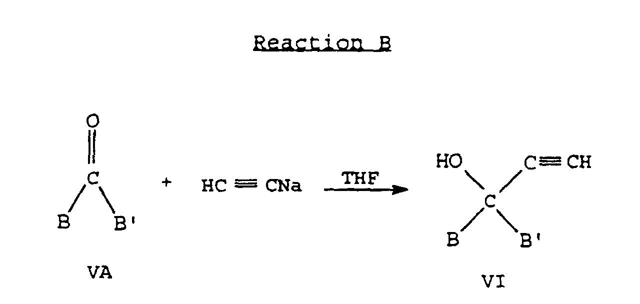

In Reaction C, the compounds represented by graphic formulae VII and VIII are first reacted at elevated temperatures and then further reacted in the presence of a base such as potassium hydroxide to form the corresponding substituted naphthol represented by graphic formula IX. Compounds VII and VIII may each be purchased. Alternatively, compound VIII may be prepared by the method described by T. Sakamoto et al., Synthesis, pages 312-314 (1983) and by S. Takahashi et al., Synthesis, pages 627-630 (1980). Synthesis of phenyl naphthols are further described in C. Kipping et al., J. Pralet. Chem., Vol. 315, pages 887-894 (1973) and J. Bao et al., J. Amer. Chem. Soc., Vol. 118, pages 3392-3405 (1996).

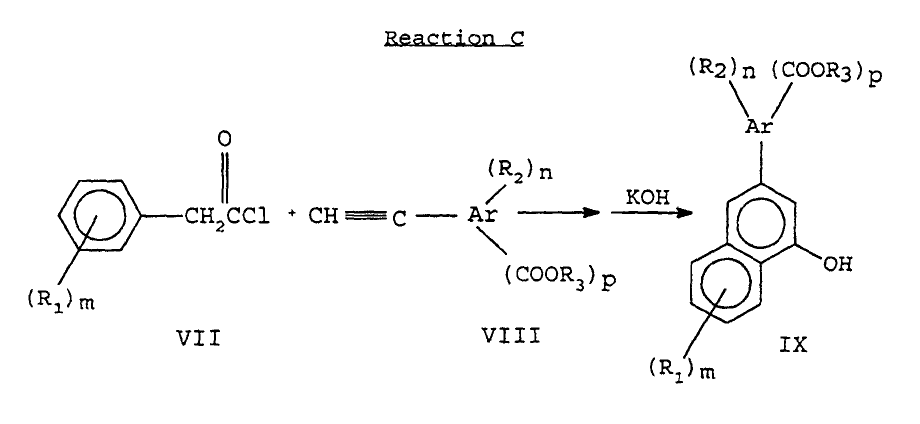

In Reaction D, the propargyl alcohol represented by graphic formula VI is coupled with the naphthol represented by graphic formula IX in the presence of an acid such as p-toluene sulfonic acid (PTSA) and in a suitable solvent such as toluene to form compounds represented by graphic formula I.

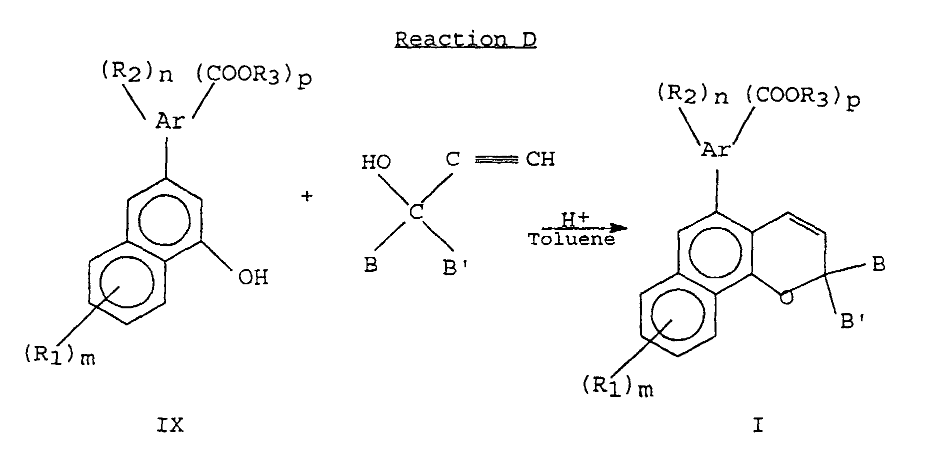

Compounds represented by graphic formula I may be used in those applications in which organic photochromic substances may be employed, such as optical lenses, e.g., vision correcting ophthalmic lenses and plano lenses, face shields, goggles, visors, camera lenses, windows, automotive windshields, aircraft and automotive transparencies, e.g., T-roofs, sidelights and backlights, plastic films and sheets, textiles and coatings, e.g., coating compositions such as paints, and verification marks on security documents, e.g., documents such as banknotes, passports and drivers' licenses for which authentication or verification of authenticity may be desired. The 5-aromatic substituted naphthopyrans represented by graphic formula I exhibit color changes from colorless to colors ranging from yellow to orange.

Examples of contemplated naphthopyran compounds within the scope of the invention include the following:
(a) 2,2-diphenyl-5-(2-methoxycarbonylphenyl)-2H-naphtho[1,2-b]pyran; and
(b) 2,2-diphenyl-5-(thien-2-yl)-2H-naphtho[1,2-b]pyran.

It is contemplated that the organic photochromic naphthopyrans of the present invention may be used alone, in combination with other naphthopyrans of the present invention, or in combination with one or more other appropriate complementary organic photochromic materials, i.e., organic photochromic compounds having at least one activated absorption maxima within the range of between about 400 and 700 nanometers, or substances containing same, and may be incorporated, e.g., dissolved or dispersed, in a polymeric organic host material used to prepare photochromic articles and which color when activated to an appropriate hue.

Other than where otherwise indicated, all numbers expressing wavelengths, quantities of ingredients or reaction conditions used herein are to be understood as modified in all instances by the term "about".

Examples of complementary organic photochromic compounds include other naphthopyrans, chromenes and oxazines, substituted 2H-phenanthro[4,3-b]pyran and 3H-phenanthro[1,2-b]pyran compounds, benzopyran compounds having substituents at the 2-position of the pyran ring including a dibenzo-fused 5 member heterocyclic compound and a substituted or unsubstituted heterocyclic ring, such as a benzothieno or benzofurano ring fused to the benzene portion of the benzopyrans, spiro(benzindoline)naphthopyrans, spiro(indoline)benzopyrans, spiro(indoline)naphthopyrans, spiro(indoline)quinopyrans, spiro(indoline)pyrans, spiro(indoline)napthoxazines, spiro(indoline)pyridobenzoxazines, spiro(benzindoline)pyridobenzoxazines, spiro(benzindoline)naphthoxazines, spiro(indoline)benzoxazines, and mixtures of such photochromic compounds. Many of such photochromic compounds are described in the open literature, e.g., U.S. Patents 3,562,172; 3,567,605; 3,578,602; 4,215,010; 4,342,668; 4,816,584; 4,818,096; 4,826,977; 4,880,667; 4,931,219; 5,066,818; 5,238,931; 5,274,132; 5,384,077; 5,405,958; 5,429,774; 5,458,814; 5,466,398; 5,514,817; 5,552,090; 5,552,091; 5,565,146; 5,573,712; 5,578,252; 5,645,767 and Japanese Patent Publication 62/195383. Spiro(indoline)pyrans are also described in the text, Techniques in Chemistry, Volume III, "Photochromism", Chapter 3, Glenn H. Brown, Editor, John Wiley and Sons, Inc., New York, 1971.

Other complementary photochromic substances contemplated are photochromic metal-dithizonates, e.g. mercury dithizonates which are described in, for example, U.S. Patent 3,361,706, fulgides and fulgimides, e.g. the 3-furyl and 3-thienyl fulgides and fulgimides which are described in U.S. Patent 4,931,220 at column 20, line 5 through column 21, line 38.

The disclosures relating to such photochromic compounds in the aforedescribed patents are incorporated herein, in toto, by reference. The photochromic articles of the present invention may contain one photochromic compound or a mixture of photochromic compounds, as desired.

Each of the photochromic substances described herein may be used in amounts (or in a ratio) such that an organic host material to which the photochromic compounds or mixture of compounds is applied or in which they are incorporated exhibits a desired resultant color, e.g., a substantially neutral color when activated with unfiltered sunlight, i.e., as near a neutral color as possible given the colors of the activated photochromic compounds. Neutral gray and neutral brown colors are preferred.

A neutral gray color exhibits a spectrum that has relatively equal absorption in the visible range between 400 and 700 nanometers. A neutral brown color exhibits a spectrum in which the absorption in the 400-550 nanometer range is moderately larger than in the 550-700 nanometer range. An alternative way of describing color is in terms of its chromaticity coordinates, which describe the qualities of a color in addition to its luminance factor, i.e., its chromaticity. In the CIE system, the chromaticity coordinates are obtained by taking the ratios of the tristimulus values to their sum, e.g., x=X/(X+Y+Z) and y=Y/(X+Y+Z). Color as described in the CIE system can be plotted on a chromaticity diagram, usually a plot of the chromaticity coordinates x and y. See pages 47-52 of Principles of Color Technology, by F. W. Billmeyer, Jr., and Max Saltzman, Second Edition, John Wiley and Sons, N.Y. (1981). As used herein, a near neutral color is one in which the chromaticity coordinate values of "x" and "y" for the color are within the following ranges (D65 illuminant): x = 0.260 to 0.400, y = 0.280 to 0.400 following activation to 40 percent luminous transmission by exposure to solar radiation (Air Mass 1 or 2).

The amount of photochromic substance or composition containing same applied to or incorporated into a host material is not critical provided that a sufficient amount is used to produce a photochromic effect discernible to the naked eye upon activation. Generally such amount can be described as a photochromic amount. The particular amount used depends often upon the intensity of color desired upon irradiation thereof and upon the method used to incorporate or apply the photochromic substances. Typically, the more photochromic substance applied or incorporated, the greater is the color intensity up to a certain limit.

The relative amounts of the aforesaid photochromic compounds used will vary and depend in part upon the relative intensities of the color of the activated species of such compounds, and the ultimate color desired. Generally, the amount of total photochromic substance incorporated into or applied to a photochromic optical host material may range from 0.05 to 1.0, e.g., from 0.1 to 0.45, milligrams per square centimeter of surface to which the photochromic substance(s) is incorporated or applied.

The photochromic substances of the present invention may be applied to or incorporated into a host material such as a polymeric organic host material by various methods described in the art. Such methods include dissolving or dispersing the photochromic substance within the host material, e.g., casting it in place by adding the photochromic substance to the monomeric host material prior to polymerization; imbibition of the photochromic substance into the host material by immersion of the host material in a hot solution of the photochromic substance or by thermal transfer; providing the photochromic substance as a separate layer between adjacent layers of the host material, e.g., as a part of a polymeric film; and applying the photochromic substance as part of a coating or film placed on the surface of the host material. The term "imbibition" or "imbibe" is intended to mean and include permeation of the photochromic substance alone into the host material, solvent assisted transfer of the photochromic substance into a porous polymer, vapor phase transfer, and other such transfer mechanisms.

Compatible (chemically and color-wise) tints, i.e., dyes, may be applied to the host material to achieve a more aesthetic result, for medical reasons, or for reasons of fashion. The particular dye selected will vary and depend on the aforesaid need and result to be achieved. In one embodiment, the dye may be selected to complement the color resulting from the activated photochromic substances, e.g., to achieve a more neutral color or absorb a particular wavelength of incident light. In another embodiment, the dye may be selected to provide a desired hue to the host matrix when the photochromic substances is in an unactivated state.

The host material will usually be transparent, but may be translucent or even opaque. The host material need only be transparent to that portion of the electromagnetic spectrum, which activates the photochromic substance, i.e., that wavelength of ultraviolet (UV) light that produces the open form of the substance and that portion of the visible spectrum that includes the absorption maximum wavelength of the substance in its UV activated form, i.e., the open form. Preferably, the host color should not be such that it masks the color of the activated form of the photochromic substance, i.e., so the change in color is readily apparent to the observer. More preferably, the host material article is a solid transparent or optically clear material, e.g., materials suitable for optical applications, such as plano and ophthalmic lenses, windows, automotive transparencies, e.g., windshields, aircraft transparencies, plastic sheeting, polymeric films, etc.

The photochromic compounds of the present invention may be present in an organic solvent or an organic polymeric host. The organic solvent may be selected from the group consisting of benzene, toluene, methyl ethyl ketone, acetone, ethanol, tetrahydrofurfuryl alcohol, N-methyl pyrrolidinone, 2-methoxyethyl ether, xylene, cyclohexane, 3-methyl cyclohexanone, ethyl acetate, tetrahydrofuran, methanol, methyl propinate, ethylene glycol and mixtures thereof. Preferably, the organic solvent is selected from the group consisting of acetone, ethanol, tetrahydrofurfuryl alcohol, 2-methoxyethyl ether, 3-methyl cyclohexanone, N-methyl pyrrolidinone and mixtures thereof.

Preferably, the organic polymeric host material is a solid transparent or optically clear material, e.g., materials suitable for optical applications, such as piano and ophthalmic lenses, windows, automotive transparencies, e.g., windshields, aircraft transparencies, plastic sheeting, polymeric films, etc.

Examples of polymeric organic host materials are polymers prepared from individual monomers or mixtures of monomers selected from the following groups:
(a) diacrylate or dimethacrylate compounds represented by graphic formula X: 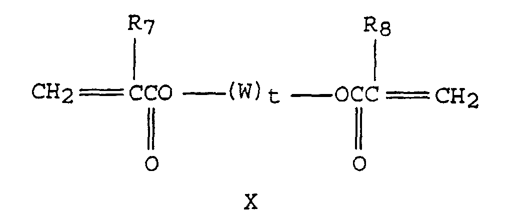 wherein R₇ and R₈ may be the same or different and are hydrogen or methyl, and W is (CH₂) and t is an integer of from 1 to 20;
(b) diacrylate or dimethacrylate compounds represented by graphic formula XI: 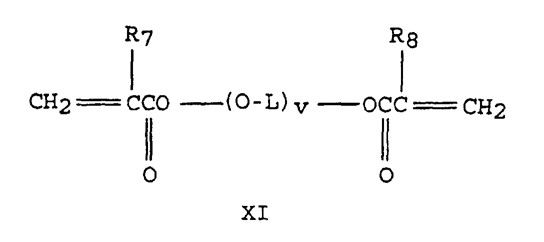 wherein L is CH₂CR₈R₉, R₉ is hydrogen or methylene and v is an integer of from 1 to 50; and
(c) an acrylate or a methacrylate compound having an epoxy group represented by graphic formula XII: 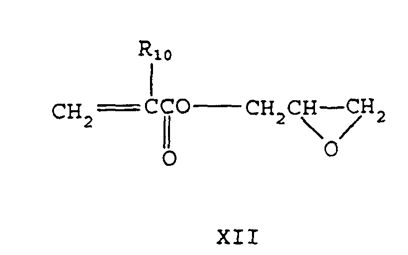
wherein R₁₀ is hydrogen or methyl.

In graphic formulae X, XI and XII, like letters used with respect to the definitions of different substituents have the same meaning.

Examples of diacrylate or dimethacrylate compounds represented by graphic formulae X and XI include diethylene glycol dimethacrylate, triethylene glycol dimethacrylate, etc., butanediol dimethacrylate and poly(oxyalkylene dimethacrylates), e.g., polyethylene glycol (600) dimethacrylate. Examples of acrylate or methacrylate compounds represented by graphic formula XII include glycidyl acrylate and glycidyl methacrylate.

Further examples of polymeric organic host materials which may be used with the photochromic compounds described herein include: polymers, i.e., homopolymers and copolymers, of the monomers and mixtures of monomers represented by graphic formulae X, XI and XII, bis(allyl carbonate) monomers, diisopropenyl benzene monomers, ethoxylated bisphenol A dimethacrylate monomers, ethylene glycol bismethacrylate monomers, poly(ethylene glycol) bismethacrylate monomers, ethoxylated phenol bismethacrylate monomers, alkoxylated polyhydric alcohol polyacrylate monomers, such as ethoxylated trimethylol propane triacrylate monomers, urethane acrylate monomers, such as those described in U.S. Patent 5,373,033, and vinylbenzene monomers, such as those described in U.S. Patent 5,475,074 and styrene; polymers, i.e., homopolymers and copolymers, of polyfunctional, e.g., mono-, di- or multi-functional, acrylate and/or methacrylate monomers, poly(C₁-C₁₂ alkyl methacrylates), such as poly(methyl methacrylate), poly(alkoxylated phenol methacrylates), cellulose acetate, cellulose triacetate, cellulose acetate propionate, cellulose acetate butyrate, poly(vinyl acetate), poly(vinyl alcohol), poly(vinyl chloride), poly(vinylidene chloride), polyurethanes, thermoplastic polycarbonates, polyesters, poly(ethylene terephthalate), polystyrene, poly(alpha methylstyrene), copoly(styrene-methyl methacrylate), copoly(styrene-acrylonitrile), polyvinylbutyral and polymers, i.e., homopolymers and copolymers, of diallylidene pentaerythritol, particularly copolymers with polyol (allyl carbonate) monomers, e.g., diethylene glycol bis(allyl carbonate), and acrylate monomers, e.g., ethyl acrylate, butyl acrylate.

Transparent copolymers and blends of transparent polymers are also suitable as host materials. Preferably, the host material is an optically clear polymerized organic material prepared from a thermoplastic polycarbonate resin, such as the carbonate-linked resin derived from bisphenol A and phosgene, which is sold under the trademark, LEXAN; a polyester, such as the material sold under the trademark, MYLAR; a poly(methyl methacrylate), such as the material sold under the trademark, PLEXIGLAS; polymerizates of a polyol(allyl carbonate) monomer, especially diethylene glycol bis(allyl carbonate), which monomer is sold under the trademark CR-39, and polymerizates of copolymers of a polyol (allyl carbonate), e.g., diethylene glycol bis(allyl carbonate), with other copolymerizable monomeric materials, such as copolymers with vinyl acetate, e.g., copolymers of from 80-90 percent diethylene glycol bis(allyl carbonate) and 10-20 percent vinyl acetate, particularly 80-85 percent of the bis(allyl carbonate) and 15-20 percent vinyl acetate, and copolymers with a polyurethane having terminal diacrylate functionality, as described in U.S. patents 4,360,653 and 4,994,208; and copolymers with aliphatic urethanes, the terminal portion of which contain allyl or acrylyl functional groups, as described in U.S. Patent 5,200,483; poly(vinyl acetate), polyvinylbutyral, polyurethane, polymers of members of the group consisting of diethylene glycol dimethacrylate monomers, diisopropenyl benzene monomers, ethoxylated bisphenol A dimethacrylate monomers, ethylene glycol bismethacrylate monomers, poly(ethylene glycol) bismethacrylate monomers, ethoxylated phenol bismethacrylate monomers and ethoxylated trimethylol propane triacrylate monomers; cellulose acetate, cellulose propionate, cellulose butyrate, cellulose acetate butyrate, polystyrene and copolymers of styrene with methyl methacrylate, vinyl acetate and acrylonitrile.

More particularly, contemplated is use of the photochromic naphthopyrans of the present invention with optical organic resin monomers used to produce optically clear polymerizates, i.e., materials suitable for optical applications, such as for example plano and ophthalmic lenses, windows, and automotive transparencies. Such optically clear polymerizates may have a refractive index that may range from about 1.48 to about 1.75, e.g., from about 1.495 to about 1.66. Specifically contemplated are optical resins sold by PPG Industries, Inc. under the CR- designation, e.g., CR-307 and CR-407.

The present invention is more particularly described in the following examples which are intended as illustrative only, since numerous modifications and variations therein will be apparent to those skilled in the art.

### EXAMPLE 1

### Step 1

Phenyl acetyl chloride (20 milliliters (mL)) and phenyl acetylene (10 mL) were mixed in a reaction flask equipped with a magnetic stirrer, oil bath and a condenser. The mixture was maintained at a temperature of 180°C and stirred overnight. After cooling to room temperature, potassium hydroxide (5 grams), water (10 mL) and methanol (200 mL) were added to the reaction flask and the reaction mixture was stirred overnight at room temperature. The methanol was removed under vacuum and the remaining black residue was dissolved in a 10 weight percent solution of potassium hydroxide (150 mL). The resulting solution was extracted with ether three times. The remaining aqueous portion was acidified and extracted with ether three times. The ether extracts were combined, washed with sodium bicarbonate solution three times and dried over anhydrous sodium sulfate. The solvent, methanol, was removed on a rotary evaporator to yield 11.0 grams of product which crystallized at room temperature. A nuclear magnetic resonance (NMR) spectrum showed the product to have a structure consistent with 3-phenyl-1-naphthol.

### Step 2

3-Phenyl-1-naphthol (4.4 grams) from Step 1, 1,1-diphenylpropargyl alcohol (4.6 grams), p-toluene sulfonic acid (0.2 gram) and toluene (100 mL) were mixed in a reaction flask and stirred for one hour at room temperature. The reaction mixture was heated to 50-60°C and stirred for another two hours. The resulting mixture was washed with water twice and dried over anhydrous sodium sulfate. The solvent, toluene, was removed using a rotary evaporator. The resulting product was purified on a silica gel column (using hexane as the eluant) to yield 3.7 grams of a crystalline product having a melting point of 150-151°C. An NMR spectrum showed the product to have a structure consistent with 2,2-diphenyl-5-phenyl-2H-naphtho[1,2-b]pyran.

### EXAMPLE 2

### PART A

Testing was done with the photochromic compound described in Example 1 in the following manner. A quantity of photochromic compound calculated to yield a 1.5 x 10⁻³ molal solution was added to a flask containing 50 grams of a monomer blend of 4 parts ethoxylated bisphenol A dimethacrylate (BPA 2EO DMA), 1 part poly(ethylene glycol) 600 dimethacrylate, and 0.033 weight percent 2,2'-azobis(2-methyl propionitrile) (AIBN). The photochromic compound was dissolved into the monomer blend by stirring and gentle heating, if necessary. After a clear solution was obtained, it was poured into a flat sheet mold having the interior dimensions of 2.2 mm x 6 inches (15.24 cm) x 6 inches (15.24 cm). The mold was sealed and placed in a horizontal air flow, programmable oven programmed to increase the temperature from 40°C to 95°C over a 5 hour interval, hold the temperature at 95°C for 3 hours, lower it to 60°C over a 2 hour interval and then hold at 60°C for 16 hours. After the mold was opened, the polymer sheet was cut using a diamond blade saw into 2 inch (5.1 centimeters) test squares.

### Part B

The photochromic test squares prepared in Part A were tested for photochromic response on an optical bench. Prior to testing on the optical bench, the photochromic test squares were exposed to 365 nanometer ultraviolet light for about 15 minutes to activate the photochromic compounds and then placed in a 76°C oven for about 15 minutes to bleach or inactivate the photochromic compounds. The test squares were then cooled to room temperature, exposed to fluorescent room lighting for at least 2 hours and then kept covered for at least 2 hours prior to testing on an optical bench maintained at 72°F (22.2°C). The bench was fitted with a 150 watt Xenon arc lamp, a remote controlled shutter, a copper sulfate bath acting as a heat sink for the arc lamp, a Schott WG-320 nm cut-off filter which removes short wavelength radiation; neutral density filter(s) and a sample holder in which the square to be tested was inserted. The power output of the optical bench, i.e., the dosage of light that the sample lens would be exposed to, was calibrated with a photochromic test square used as a reference standard. This resulted in a power output ranging from 0.15 to 0.20 milliWatts per square centimeter (mW/cm²). Measurement of the power output was made using a GRASEBY Optronics Model S-371 portable photometer (Serial #21536) with a UV-A detector (Serial #22411) or comparable equipment. The UV-A detector was placed into the sample holder and the light output was measured. Adjustments to the power output were made by increasing or decreasing the lamp wattage or by adding or removing neutral density filters in the light path.

A monitoring, collimated beam of light from a tungsten lamp was passed through the square at a small angle (approximately 30°) normal to the square. After passing through the square, the light from the tungsten lamp was directed to a detector through Spectral Energy Corp. GM-200 monochromator set at the previously determined visible lambda max of the photochromic compound being measured. The output signals from the detector were processed by a radiometer.

Change in optical density (ΔOD) was determined by inserting a test square in the bleached state into the sample holder, adjusting the transmittance scale to 100%, opening the shutter from the Xenon lamp to provide ultraviolet radiation to change the test square from the bleached state to an activated (i.e., darkened) state, measuring the transmittance in the activated state, and calculating the change in optical density according to the formula: ΔOD=log(100/%Ta), where %Ta is the percent transmittance in the activated state and the logarithm is to the base 10.

The optical properties of the photochromic compound in the test squares are reported in Table 1. The Δ OD/Min, which represents the sensitivity of the photochromic compound's response to UV light, was measured over the first five (5) seconds of UV exposure, then expressed on a per minute basis. The saturation optical density (Δ OD@ Saturation) was taken under identical conditions as the Δ OD/Min, except UV exposure was continued for 15 minutes. The lambda max (Vis) is the wavelength in nanometers (nm) in the visible spectrum at which the maximum absorption of the activated (colored) form of the photochromic compound in a test square occurs. The lambda max (Vis) wavelength was determined by testing the photochromic test square polymerizates of Part A in a Varian Cary 3 UV-Visible spectrophotometer. The lambda (λ) max (UV) is the wavelength in nanometers in the ultraviolet range closest to the visible spectrum at which the absorption of the photochromic compound occurs. This absorption was also determined with the same spectrophotometer. The Bleach Rate (T 1/2) is the time interval in seconds for the absorbance of the activated form of the photochromic compound in the test squares to reach one half the highest absorbance at room temperature (72°F, 22.2°C) after removal of the source of activating light.

**TABLE 1**

| | |
|---|---|
| (λ) max (VIS) | 489 nm |
| (λ) max (UV) | 348 nm |
| ΔOD/MIN Sensitivity | 0.12 |
| ΔOD@ Saturation | 0.27 |
| Bleach Rate (T 1/2) | 120 seconds |

The present invention has been described with reference to specific details of particular embodiments thereof. It is not intended that such details be regarded as limitations upon the scope of the invention except insofar as to the extent that they are included in the accompanying claims.

## Claims

1. A naphthopyran compound represented by the following graphic formula: 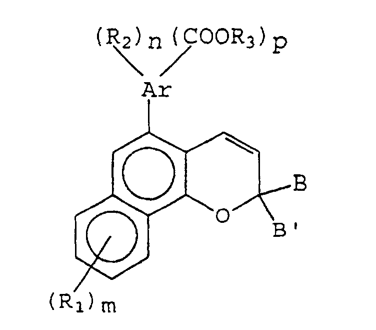 wherein,
(a) each R₁ is C₁-C₆ alkyl, C₁-C₆ alkoxy, bromo, chloro or fluoro and m is the integer 0, 1, 2 or 3;
(b) Ar is selected from the group consisting of:
(i) the aryl groups, phenyl and naphthyl; and
(ii) the aromatic groups, furanyl, benzofuran-2-yl, benzofuran-3-yl, thienyl, benzothien-2-yl, benzothien-3-yl, dibenzofuranyl, dibenzothienyl and fluorenyl;
(c) each R₂ is selected from the group consisting of aryl, mono(C₁-C₆)alkoxyaryl, di(C₁-C₆)alkoxyaryl, mono(C₁-C₆)alkylaryl, di(C₁-C₆)alkylaryl, bromoaryl, chloroaryl, fluoroaryl, C₃-C₇ cycloalkylaryl, C₃-C₇ cycloalkyl, C₃-C₇ cycloalkyloxy, C₃-C₇ cycloalkyloxy(C₁-C₆)alkyl, C₃-C₇ cycloalkyloxy(C₁-C₆)alkoxy, aryl(C₁-C₆)alkyl, aryl(C₁-C₆)alkoxy, aryloxy, aryloxy(C₁-C₆)alkyl, aryloxy(C₁-C₆)alkoxy, mono- and di(C₁-C₆)alkylaryl(C₁-C₆)alkyl, mono- and di (C₁-C₆) alkoxyaryl (C₁-C₆)alkyl, mono- and di (C₁-C₆) alkylaryl (C₁-C₆)alkoxy, mono- and di (C₁-C₆) alkoxyaryl (C₁-C₆) alkoxy, C₁-C₆ alkyl, C₁-C₆ bromoalkyl, C₁-C₆ chloroalkyl, C₁-C₆ fluoroalkyl, C₁-C₆ alkoxy, mono(C₁-C₆)alkoxy(C₁-C₄)alkyl, bromo, chloro and fluoro and n is the integer 0, 1, 2, or 3;
(d) R₃ is selected from the group consisting of C₁-C₄ alkyl, phenyl, mono(C₁-C₄)alkyl substituted phenyl, mono(C₁-C₄)alkoxy substituted phenyl, phenyl(C₁-C₂)alkyl, mono(C₁-C₄)alkyl substituted phenyl(C₁-C₂)alkyl, mono(C₁-C₄)alkoxy substituted phenyl(C₁-C₂)alkyl, mono(C₁-C₄)alkoxy(C₂-C₃)alkyl, or C₁-C₄ haloalkyl, and p is the integer 0, 1 or 2, the sum of n and p being 0, 1, 2 or 3 and wherein when p is 2, n is 0; and
(e) B and B' are each selected from the group consisting of:
(i) the unsubstituted, mono-, di- and trisubstituted aryl groups, phenyl and naphthyl;
(ii) the unsubstituted, mono- and di-substituted hetroaromatic groups pyridyl, furanyl, benzofuran-2-yl, benzofuran-3-yl, thienyl, benzothien-2-yl, benzothien-3-yl, dibenzofuranyl, dibenzothienyl, carbazolyl and fluorenyl, each of said aryl and heteroaromatic substituents in (e)(i) and (ii) being selected from the group consisting of hydroxy, aryl, mono(C₁-C₆)alkoxyaryl, di(C₁-C₆)alkoxyaryl, mono(C₁-C₆)alkylaryl, di(C₁-C₆)alkylaryl, bromoaryl, chloroaryl, fluoroaryl, C₃-C₇ cycloalkylaryl, C₃-C₇ cycloalkyl, C₃-C₇ cycloalkyloxy, C₃-C₇ cycloalkyloxy(C₁-C₆)alkyl, C₃-C₇ cycloalkyloxy(C₁-C₆)alkoxy, aryl(C₁-C₆)alkyl, aryl(C₁-C₆)alkoxy, aryloxy, aryloxy(C₁-C₆)alkyl, aryloxy(C₁-C₆)alkoxy, mono- and di-(C₁-C₆)alkylaryl(C₁-C₆)alkyl, mono- and di-(C₁-C₆)alkoxyaryl(C₁-C₆)alkyl, mono- and di-(C₁-C₆)alkylaryl(C₁-C₆)alkoxy, mono- and di-(C₁-C₆)alkoxyaryl(C₁-C₆)alkoxy, amino, mono(C₁-C₆)alkylamino, di(C₁-C₆)alkylamino, diarylamino, N-(C₁-C₆)alkylpiperazino, N-arylpiperazino, aziridino, indolino, piperidino, arylpiperidino, morpholino, thiomorpholino, tetrahydroquinolino, tetrahydroisoquinolino, pyrryl, C₁-C₆ alkyl, C₁-C₆ bromoalkyl, C₁-C₆ chloroalkyl, C₁-C₆ fluoroalkyl, C₁-C₆ alkoxy, mono (C₁-C₆) alkoxy (C₁-C₄) alkyl, acryloxy, methacryloxy, bromo, chloro and fluoro;
(iii) the groups represented by the following graphic formulae: 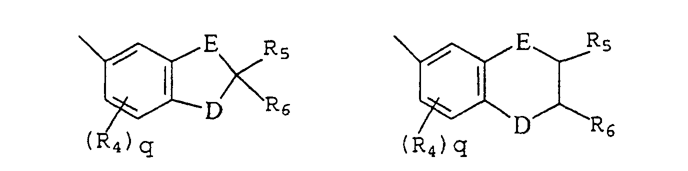 wherein E is carbon or oxygen and D is oxygen or substituted nitrogen, provided that when D is substituted nitrogen, E is carbon, said nitrogen substituents being selected from the group consisting of hydrogen, C₁-C₆ alkyl and C₂-C₆ acyl; each R₄ is C₁-C₆ alkyl, C₁-C₆ alkoxy, hydroxy, bromo, chloro or fluoro; R₅ and R₆ are each hydrogen or C₁-C₆ alkyl; and q is the integer 0, 1 or 2;
(iv) C₁-C₆ alkyl, C₁-C₆ bromoalkyl, C₁-C₆ chloroalkyl, C₁-C₆ fluoroalkyl, C₁-C₆ alkoxy(C₁-C₄)alkyl; and
(v) the group represented by the following graphic formula: 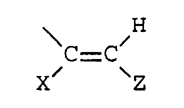
wherein X is hydrogen or C₁-C₄ alkyl and Z is selected from the unsubstituted, mono-, and di-substituted members of the group consisting of naphthyl, phenyl, furanyl and thienyl, each of said group substituents being C₁-C₄ alkyl, C₁-C₄ alkoxy, bromo, fluoro or chloro.

2. The naphthopyran of claim 1 wherein,
(a) each R₁ is C₁-C₄ alkyl, C₁-C₄ alkoxy, chloro or fluoro, and m is the integer 0, 1 or 2;
(b) Ar is phenyl or thienyl;
(c) each R₂ is selected from the group consisting of aryl, aryloxy, aryl(C₁-C₃)alkyl, C₁-C₃ alkyl, C₁-C₃ chloroalkyl, C₁-C₃ fluoroalkyl, C₁-C₃ alkoxy, mono(C₁-C₃)alkoxy(C₁-C₃)alkyl, fluoro and chloro and n is the integer 0, 1 or 2;
(d) R₃ is C₁-C₃ alkyl and p is the integer 0 or 1, wherein when p is 1, n is 0; and
(e) B and B' are each selected from the group consisting of:
(i) phenyl, mono-substituted phenyl and di-substituted phenyl;
(ii) the unsubstituted, mono-substituted and di-substituted heteroaromatic groups furanyl, benzofuran-2-yl, thienyl, benzothien-2-yl, dibenzofuran-2-yl, and dibenzothien-2-yl, each of said phenyl and heteroaromatic substituents in (e)(i) and (ii) being selected from the group consisting of hydroxy, aryl, aryloxy, aryl(C₁-C₃)alkyl, amino, mono(C₁-C₃)alkylamino, di(C₁-C₃)alkylamino, N-(C₁-C₃)alkylpiperazino, indolino, piperidino, morpholino, pyrryl, C₁-C₃ alkyl, C₁-C₃ chloroalkyl, C₁-C₃ fluoroalkyl, C₁-C₃ alkoxy, mono(C₁-C₃)alkoxy(C₁-C₃)alkyl, fluoro and chloro;
(iii) the groups represented by the following graphic formulae: 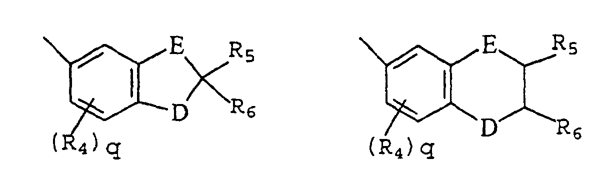 wherein E is carbon and D is oxygen, R₄ is C₁-C₃ alkyl or C₁-C₃ alkoxy, R₅ and R₆ are each hydrogen or C₁-C₄ alkyl; and q is the integer 0 or 1;
(iv) C₁-C₄ alkyl; and
(v) the group represented by the following graphic formula: 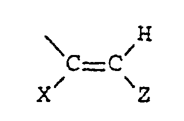 wherein X is hydrogen or methyl and Z is phenyl or mono-substituted phenyl, said phenyl substituent being selected from the group consisting of C₁-C₃ alkyl, C₁-C₃ alkoxy and fluoro.

3. The naphthopyran of claim 2 wherein:
(a) R₁ is C₁-C₃ alkyl or C₁-C₃ alkoxy, and m is the integer 0 or 1;
(b) Ar is phenyl or thienyl;
(c) R₂ is C₁-C₃ alkyl, C₁-C₃ alkoxy, aryl, fluoro or chloro, and n is the integer 0 or 1;
(d) R₃ is C₁-C₃ alkyl and p is the integer 0 or 1, wherein when p is 1, n is 0; and
(e) B and B' are each selected from the group consisting of:
(i) phenyl, mono-substituted and di-substituted phenyl;
(ii) the unsubstituted, mono-, and di-substituted heteroaromatic groups furanyl, benzofuran-2-yl, thienyl and benzothien-2-yl, each of said phenyl and heteroaromatic substituents in (e)(i) and (ii) being selected from the group consisting of hydroxy, C₁-C₃ alkyl, C₁-C₃ alkoxy, aryl, indolino, fluoro and chloro; and
(iii) the group represented by the following graphic formula: 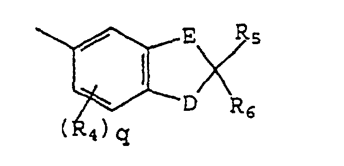
wherein E is carbon and D is oxygen, R₄ is C₁-C₃ alkyl or C₁-C₃ alkoxy, R₅ and R₆ are each hydrogen or C₁-C₃ alkyl, and q is the integer 0 or 1.

4. A naphthopyran compound selected from the group consisting of:
(a) 2,2-diphenyl-5-(2-methyoxycarbonylphenyl)-2H-naphtho [1,2-b]pyran; and
(b) 2,2-diphenyl-5-(thien-2-yl)-2H-naphtho[1,2-b]pyran.

5. A photochromic article comprising a polymeric organic host material and a photochromic amount of the naphthopyran compound of any of claims 1-4.

6. A photochromic article comprising, in combination, a solid transparent polymeric organic host material, and a photochromic amount of each of (a) at least one naphthopyran compound of any of claim 1-4, and (b) at least one other organic photochromic compound having at least one activated absorption maxima within the range of between 400 and 700 nanometers.

7. The photochromic article of claim 6 wherein the organic photochromic compound (b) is selected from the group consisting of other naphthopyrans, chromenes. oxazines, metal-dithizonates, fulgides and fulgimides.

8. The photochromic article of any of claims 5-7 wherein the total amount of photochromic compound present is from 0.05 to 1.0 milligram per square centimeter of organic host material surface to which the photochromic substance(s) is incorporated or applied.

9. The photochromic article of any of claims 5-8 wherein the host material is a polymerizate of an optical organic resin monomer.

10. The photochromic article of claim 9 wherein the refractive index of the polymerizate is from 1.48 to 1.75.

11. The photochromic article of claim 10 wherein the refractive index of the polymerizate is from 1.495 to 1.66.

12. The photochromic article of any of claims 5-8 wherein the polymeric organic host material is selected from the group consisting of poly(C₁-C₁₂ alkyl methacrylates), poly(oxyalkylene dimethacrylates), poly(alkoxylated phenol methacrylates), cellulose acetate, cellulose triacetate, cellulose acetate propionate, cellulose acetate butyrate, poly(vinyl acetate), poly(vinyl alcohol), poly(vinyl chloride), poly(vinylidene chloride), thermoplastic polycarbonates, polyesters, polyurethanes, poly(ethylene terephthalate), polystyrene, poly(alpha methylstyrene), copoly(styrene-methylmethacrylate), copoly(styrene-acrylonitrile), polyvinylbutyral and polymers of members of the group consisting of bis(allyl carbonate) monomers, polyfunctional acrylate monomers, polyfunctional methacrylate monomers, diethylene glycol dimethacrylate monomers, diisopropenyl benzene monomers, ethoxylated bisphenol A dimethacrylate monomers, ethylene glycol bismethacrylate monomers, poly(ethylene glycol) bismethacrylate monomers, ethoxylated phenol bismethacrylate monomers, alkoxylated polyhydric alcohol acrylate monomers, styrene monomers, urethane acrylate monomers, glycidyl acrylate monomers, glycidyl methacrylate monomers and diallylidene pentaerythritol monomers.

13. The photochromic article of claim 12 wherein the polymeric organic host material is a solid transparent polymer selected from the group consisting of poly(methyl methacrylate), poly(ethylene glycol bismethacrylate), poly(ethoxylated bisphenol A dimethacrylate), thermoplastic polycarbonate, poly(vinyl acetate), polyvinylbutyral, polyurethane and polymers of members of the group consisting of diethylene glycol bis(allyl carbonate) monomers, diethylene glycol dimethacrylate monomers, ethoxylated phenol bismethacrylate monomers, diisopropenyl benzene monomers and ethoxylated trimethylol propane triacrylate monomers.

14. The photochromic article of any of claims 5-13 wherein said transparent polymeric organic host material is an optical element.

15. The photochromic article of claim 14 wherein said optical element is a lens.

## Patentansprüche

1. Naphthopyranverbindung, die durch die folgende grafische Formel wiedergegeben ist: 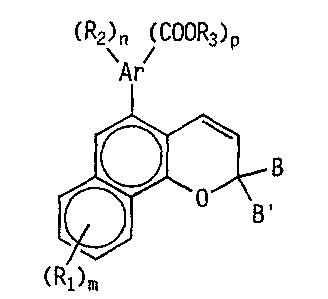 worin
(a) jedes R₁ C₁- bis C₆-Alkyl , C₁- bis C₆-Alkoxy, Brom, Chlor oder Fluor ist und m die ganze Zahl 0, 1, 2 oder 3 ist,
(b) Ar ausgewählt ist aus der Gruppe bestehend aus
(i) den Arylgruppen Phenyl und Naphthyl und
(ii) den aromatischen Gruppen Furanyl, Benzofuran-2-yl, Benzofuran-3-yl, Thienyl, Benzothien-2-yl, Benzothien-3-yl, Dibenzofuranyl, Dibenzothienyl und Fluorenyl,
(c) jedes R₂ ausgewählt ist aus der Gruppe bestehend aus Aryl, Mono(C₁- bis C₆-)alkoxyaryl, Di(C₁- bis C₆-)alkoxyaryl, Mono(C₁-bis C₆-)alkylaryl, Di(C₁- bis C₆-)alkylaryl, Bromaryl, Chloraryl, Fluoraryl, C₃- bis C₇-Cycloalkylaryl, C₃- bis C₇-Cycloalkyl, C₃- bis C₇-Cycloalkyloxy, C₃- bis C₇-Cycloalkyloxy(C₁- bis C₆-)alkyl, C₃- bis C₇-Cycloalkyloxy(C₁- bis C₆-)alkoxy, Aryl(C₁- bis C₆-)alkyl, Aryl(C₁- bis C₆-)alkoxy, Aryloxy, Aryloxy(C₁- bis C₆-)alkyl, Aryloxy(C₁- bis C₆-)alkoxy, Mono- und Di(C₁- bis C₆-)alkylaryl(C₁- bis C₆-)alkyl, Mono- und Di(C₁- bis C₆-)alkoxyaryl(C₁- bis C₆-)alkyl, Mono- und Di(C₁- bis C₆-)alkylaryl(C₁- bis C₆-)alkoxy, Mono- und Di(C₁- bis C₆-)-alkoxyaryl(C₁- bis C₆-)alkoxy, C₁- bis C₆-Alkyl, C₁- bis C₆-Bromalkyl, C₁- bis C₆-Chloralkyl, C₁- bis C₆-Fluoralkyl, C₁- bis C₆-Alkoxy, Mono(C₁- bis C₆-)alkoxy(C₁- bis C₄-)alkyl, Brom, Chlor und Fluor und n die ganze Zahl 0, 1, 2 oder 3 ist,
(d) R₃ ausgewählt ist aus der Gruppe bestehend aus C₁- bis C₄-Alkyl, Phenyl, mono(C₁- bis C₄-)alkylsubstituiertem Phenyl, mono(C₁- bis C₄-)alkoxysubstituiertem Phenyl, Phenyl(C₁- bis C₂-)-alkyl, mono(C₁- bis C₄-)alkylsubstituiertem Phenyl(C₁- bis C₂-)-alkyl, mono(C₁- bis C₄-)alkoxysubstituiertem Phenyl(C₁- bis C₂-)alkyl, Mono(C₁- bis C₄-)alkoxy(C₂- bis C₃-)alkyl oder C₁- bis C₄-Halogenalkyl und p die ganze Zahl 0, 1 oder 2 ist, wobei die Summe von n und p gleich 0, 1, 2 oder 3 ist, und wobei, wenn p gleich 2 ist, n gleich 0 ist, und
(e) B und B' jeweils ausgewählt sind aus der Gruppe bestehend aus:
(i) den unsubstituierten, mono-, di- und trisubstituierten Arylgruppen Phenyl und Naphthyl,
(ii) den unsubstituierten , mono- und disubstituierten heteroaromatischen Gruppen Pyridyl, Furanyl, Benzofuran-2-yl, Benzofuran-3-yl, Thienyl, Benzothien-2-yl, Benzothien-3-yl, Dibenzofuranyl, Dibenzothienyl, Carbozolyl und Fluorenyl, wobei jeder dieser Substituenten der Aryl- und heteroaromatischen Gruppen in (e)(i) und (ii) ausgewählt ist aus der Gruppe bestehend aus Hydroxy, Aryl, Mono(C₁- bis C₆-)alkoxyaryl, Di(C₁- bis C6-)-alkoxyaryl, Mono(C₁- bis C₆-)alkylaryl, Di(C₁- bis C₆-)-alkylaryl, Bromaryl, Chloraryl, Fluoraryl, C₃- bis C₇-Cycloalkylaryl, C₃- bis C₇-Cycloalkyl, C₃- bis C₇-Cycloalkyloxy, C₃- bis C₇-Cycloalkyloxy(C₁- bis C₆-)alkyl, C₃- bis C₇-Cycloalkyloxy(C₁- bis C₆-)alkoxy, Aryl(C₁- bis C₆-)alkyl, Aryl(C₁- bis C₆-)alkoxy, Aryloxy, Aryloxy(C₁-bis C₆-)alkyl, Aryloxy(C₁- bis C₆-)alkoxy, Mono- und Di(C₁- bis C₆-)alkylaryl(C₁- bis C₆-)alkyl, Mono- und Di(C₁- bis C₆-)alkoxyaryl(C₁- bis C₆-)alkyl, Mono- und Di(C₁- bis C₆-)alkylaryl(C₁- bis C₆-)alkoxy, Mono- und Di(C₁- bis C₆-)alkoxyaryl(C₁- bis C₆-)alkoxy, Amino, Mono(C₁- bis C₆-)alkylamino, Di(C₁- bis C₆-)alkylamino, Diarylamino, N-(C₁- bis C₆-)alkylpiperazino, N-Arylpiperazino, Aziridino, Indolino, Piperidino, Arylpiperidino, Morpholino, Thiomorpholino, Tetrahydrochinolino, Tetrahydroisochinolino, Pyrryl, C₁- C6-Alkyl, C₁- bis C₆-Bromalkyl, C₁- bis C₆-Chloralkyl, C₁- bis C₆-Fluoralkyl, C₁- bis C₆-Alkoxy, Mono(C₁- bis C₆-)alkoxy(C₁-bis C₄-)alkyl, Acryloxy, Methacryloxy, Brom, Chlor und Fluor,
(iii) den Gruppen, die durch die folgende grafische Formel wiedergegeben sind: 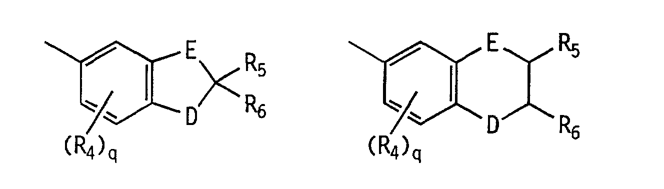 worin E Kohlenstoff oder Sauerstoff ist und D Sauerstoff oder substituierter Stickstoff ist, unter der Voraussetzung, dass, wenn D substituierter Stickstoff ist, E Kohlenstoff ist, wobei diese Stickstoffsubstituenten ausgewählt sind aus der Gruppe bestehend aus Wasserstoff, C₁- bis C₆-Alkyl und C₂- bis C₆-Acyl, wobei jedes R₄ C₁- bis C₆-Alkyl, C₁- bis C₆-Alkoxy, Hydroxy, Brom, Chlor oder Fluor ist, R₅ und R₆ jeweils Wasserstoff oder C₁- bis C₆-Alkyl sind und q die ganze Zahl 0, 1 oder 2 ist,
(iv) C₁- bis C₆-Alkyl, C₁- bis C₆-Bromalkyl, C₁- bis C₆-Chloralkyl, C₁- bis C₆-Fluoralkyl, C₁- bis C₆-Alkoxy(C₁- bis C₄-)alkyl und
(v) der Gruppe, die durch die folgende grafische Formel wiedergegeben ist: 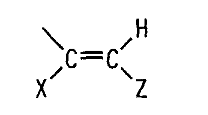
worin X Wasserstoff oder C₁- bis C₄-Alkyl ist und Z ausgewählt ist aus den unsubstituierten, mono- und disubstituierten Mitgliedern der Gruppe bestehend aus Naphthyl, Phenyl, Furanyl und Thienyl, wobei jeder dieser Gruppensubstituenten C₁- bis C₄-Alkyl, C₁- bis C₆-Alkoxy, Brom, Fluor oder Chlor ist.

2. Naphthopyran nach Anspruch 1, worin
(a) jedes R₁ C₁- bis C₄-Alkyl, C₁- bis C₄-Alkoxy, Chlor oder Fluor ist und m die ganze Zahl 0, 1 oder 2 ist,
(b) Ar Phenyl oder Thienyl ist,
(c) jedes R₂ ausgewählt ist aus der Gruppe bestehend aus Aryl, Aryloxy, Aryl(C₁- bis C₃-)alkyl, C₁- bis C₃-Alkyl, C₁- bis C₃-Chloralkyl, C₁- bis C₃-Fluoralkyl, C₁- bis C₃-Alkoxy, Mono(C₁-bis C₃-)alkoxy(C₁- bis C₃-)alkyl, Fluor und Chlor und n die ganze Zahl 0, 1 oder 2 ist,
(d) R₃ C₁- bis C₃-Alkyl ist und p die ganze Zahl 0 oder 1, wobei, wenn p gleich 1 ist, n gleich 0 ist, und
(e) B und B' jeweils ausgewählt sind aus der Gruppe bestehend aus:
(i) Phenyl, monosubstituiertem Phenyl und disubstituiertem Phenyl,
(ii) den unsubstituierten, monosubstituierten und disubstituierten heteroaromatischen Gruppen Furanyl, Benzofuran-2-yl, Thienyl, Benzothien-2-yl, Dibenzofuran-2-yl und Dibenzothien-2-yl, wobei jeder dieser Substituenten der Phenyl- und heteroaromatischen Gruppen in (e)(i) und (ii) ausgewählt ist aus der Gruppe bestehend aus Hydroxy, Aryl, Aryloxy, Aryl(C₁- bis C₃-)-alkyl, Amino, Mono(C₁- bis C₃-)alkylamino, Di(C₁- bis C₃-)alkylamino, N-(C₁- bis C₃-)alkylpiperazino, Indolino, Piperidino, Morpholino, Pyrryl, C₁- bis C₃-Alkyl, C₁- bis C₃-Chloralkyl, C₁- bis C₃-Fluoralkyl, C₁- bis C₃-Alkoxy, Hono(C₁- bis C₃-)alkoxy(C₁- bis C₃-)alkyl, Fluor und Chlor,
(iii) den Gruppen, die durch die folgenden grafischen Formeln wiedergegeben sind: 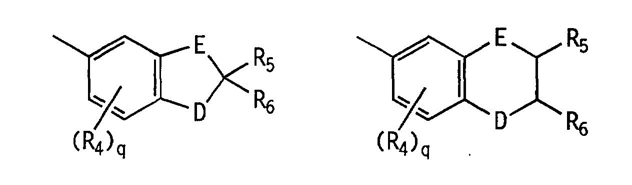 worin E Kohlenstoff und D Sauerstoff ist, R₄ C₁- bis C₃-Alkyl oder C₁- bis C₃-Alkoxy ist, R₅ und R₆ jeweils Wasserstoff oder C₁- bis C₄-Alkyl sind und q die ganze Zahl 0 oder 1 ist,
(iv) C₁- bis C₄-Alkyl und
(v) der Gruppe, die durch die folgende grafische Formel wiedergegeben ist: 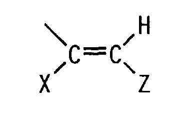 worin X Wasserstoff oder Methyl ist und Z Phenyl oder monosubstituiertes Phenyl ist, wobei diese Phenylsubstituenten ausgewählt sind aus der Gruppe bestehend aus C₁- bis C₃-Alkyl, C₁- bis C₃-Alkoxy und Fluor.

3. Naphthopyran nach Anspruch 2, worin
(a) R₁ C₁- bis C₃-Alkyl oder C₁- bis C₃-Alkoxy ist und m die ganze Zahl 0 oder 1 ist,
(b) Ar Phenyl oder Thienyl ist,
(c) R₂ C₁- bis C₃-Alkyl, C₁- bis C₃-Alkoxy, Aryl, Fluor oder Chlor ist und n die ganze Zahl 0 oder 1 ist,
(d) R₃ C₁- bis C₃-Alkyl und p die ganze Zahl 0 oder 1 ist, wobei, wenn p gleich 1 ist, n gleich 0 ist, und
(e) B und B' jeweils ausgewählt sind aus der Gruppe bestehend aus:
(i) Phenyl, monosubstituiertem und disubstituiertem Phenyl,
(ii) den unsubstituierten, mono- und disubstituierten heteroaromatischen Gruppen Furanyl, Benzofuran-2-yl, Thienyl und Benzothien-2-yl, wobei jeder dieser Substituenten der Phenyl- und heteroaromatischen Gruppen in (e)(i) und (ii) ausgewählt ist aus der Gruppe bestehend aus Hydroxy, C₁- bis C₃-Alkyl, C₁- bis C₃-Alkoxy, Aryl, Indolino, Fluor und Chlor, und
(iii) der Gruppe, die durch die folgende grafische Formel wiedergegeben ist: 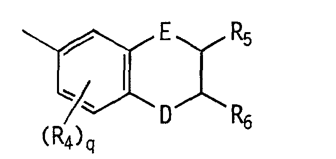
worin E Kohlenstoff und D Sauerstoff ist, R₄ C₁- bis C₃-Alkyl oder C₁- bis C₃-Alkoxy ist, R₅ und R₆ jeweils Wasserstoff oder C₁- bis C₃-Alkyl sind und q die ganze Zahl 0 oder 1 ist.

4. Naphthopyranverbindung, ausgewählt aus der Gruppe bestehend aus:
(a) 2,2-Diphenyl-5-(2-methoxycarbonylphenyl)-2H-naphtho[1,2-b]pyran und
(b) 2,2-Diphenyl-5-(thien-2-yl)-2H-naphtho[1,2-b]pyran.

5. Ein photochromer Gegenstand, der ein polymeres organisches Wirtsmaterial und eine photochrome Menge einer Naphthopyranverbindung nach einem der Ansprüche 1-4 enthält.

6. Ein photochromer Gegenstand, der in Kombination ein festes transparentes polymeres organisches Wirtsmaterial und eine photochrome Menge von jeweils (a) wenigstens einer Naphthopyranverbindung nach einem der Ansprüche 1-4 und (b) wenigstens einer anderen organischen photochromen Verbindung, die wenigstens ein aktiviertes Absorptionsmaximum innerhalb eines Bereichs zwischen 400 und 700 nm aufweist, enthält.

7. Photochromer Gegenstand nach Anspruch 6, wobei die organische photochrome Verbindung (b) ausgewählt ist aus der Gruppe bestehend aus anderen Naphthopyranen, Chromenen, Oxazinen, Metalldithizonaten, Fulgiden und Fulgimiden.

8. Photochromer Gegenstand nach einem der Ansprüche 5-7, wobei die Gesamtmenge an vorhandener photochromer Verbindung 0,05 bis 1,0 mg pro Quadratzentimeter Oberfläche des organischen Wirtsmaterials beträgt, auf die die photochrome(n) Substanz(en) eingebracht oder aufgebracht ist/sind.

9. Photochromer Gegenstand nach einem der Ansprüche 5-8, wobei das Wirtsmaterial ein Polymerisat aus einem optischen organischen Harzmonomer ist.

10. Photochromer Gegenstand nach Anspruch 9, wobei der Brechungsindex des Polymerisats 1,48 bis 1,75 ist.

11. Photochromer Gegenstand nach Anspruch 10, wobei der Brechungsindex des Polymerisats 1,495 bis 1,66 ist.

12. Photochromer Gegenstand nach einem der Ansprüche 5-8, wobei das polymere organische Wirtsmaterial ausgewählt ist aus der Gruppe bestehend aus Poly(C₁- bis C₁₂-alkylmethacrylaten), Poly-(oxyalkylendimethacrylaten), poly(alkoxylierten Phenolmethacrylaten), Celluloseacetat, Cellulosetriacetat, Celluloseacetatpropionat, Celluloseacetatbutyrat, Poly(vinylacetat), Poly(vinylalkohol), Poly(vinylchlorid), Poly(vinylidenchlorid), thermoplastischen Polycarbonaten, Polyestern, Polyurethanen, Poly(ethylenterephthalat), Polystyrol, Poly(α-methylstyrol), Copoly-(styrol -methylmethacrylat), Copoly(styrol-acrylnitril), Polyvinylbutyral und Polymeren der Mitglieder der Gruppe bestehend aus Bis(allylcarbonat)monomeren, polyfunktionellen Acrylatmonomeren, polyfunktionellen Methacrylatmonomeren, Diethylenglykoldimethacrylatmonomeren, Diisopropenylbenzolmonomeren, ethoxylierten Bisphenol-A-dimethacrylatmonomeren, Ethylenglykolbismethacrylatmonomeren, Poly(ethylenglykol)bismethacrylatmonomeren, ethoxylierten Phenolbismethacrylatmonomeren, Acrylatmonomeren alkoxylierter mehrwertiger Alkohole, Styrolmonomeren, Urethanacrylatmonomeren, Glycidylacrylatmonomeren, Glycidylmethacrylatmonomeren und Diallylidenpentaerythritolmonomeren.

13. Photochromer Gegenstand nach Anspruch 12, wobei das polymere organische Wirtsmaterial ein festes transparentes Polymer ist, ausgewählt aus der Gruppe bestehend aus Poly(methylmethacrylat), Poly-(ethylenglykolbismethacrylat), poly(ethoxyliertem Bisphenol-A-dimethacrylat), thermoplastischem Polycarbonat, Poly(vinylacetat), Polyvinylbutyral, Polyurethan und Polymeren der Mitglieder der Gruppe bestehend aus Diethylenglykolbis(allylcarbonat)monomeren, Diethylenglykoldimethacrylatmonomeren, ethoxylierten Phenolbismethacrylatmonomeren, Diisopropenylbenzolmonomeren und ethoxylierten Trimethylolpropantriacrylatmonomeren.

14. Photochromer Gegenstand nach einem der Ansprüche 5-13, wobei dieses transparente polymere organische Wirtsmaterial ein optisches Element ist.

15. Photochromer Gegenstand nach Anspruch 14, wobei dieses optische Element eine Linse ist.

## Revendications

1. Composé de naphtopyranne représenté par la formule graphique suivante : 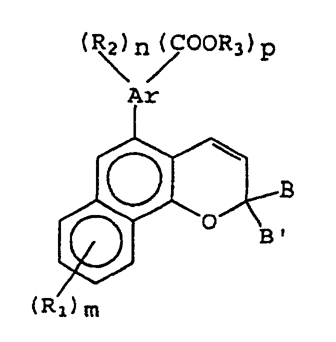 dans laquelle :
(a) chaque R₁ est un groupe alkyle en C₁-C₆, alcoxy en C₁-C₆, bromo, chloro ou fluoro et m est le nombre entier 0, 1, 2 ou 3;
(b) Ar est choisi dans le groupe comprenant :
(i) les groupes aryle, phényle et naphtyle; et
(ii) les groupes aromatiques furannyle, benzofurann-2-yle, benzofurann-3-yle, thiényle, benzothién-2-yle, benzothién-3-yle, dibenzofurannyle, dibenzothiényle et fluorényle;
(c) chaque R₂ est choisi dans le groupe comprenant les groupes aryle, monoalcoxy(C₁-C₆)aryle, dialcoxy(C₁-C₆)aryle, monoalkyl(C₁-C₆)-aryle, dialkyl(C₁-C₆)aryle, bromoaryle, chloroaryle, fluoroaryle, cycloalkylaryle en C₃-C₇, cycloakyle en C₃-C₇, cycloalkyloxy en C₃-C₇, cycloalkyloxy(C₃-C₇) alkyle en C₁-C₆, cycloalkyloxy-(C₃-C₇)alcoxy en C₁-C₆, arylalkyle en C₁-C₆, arylalcoxy en C₁-C₆, aryloxy, aryloxyalkyle en C₁-C₆, aryloxyalcoxy en C₁-C₆, mono- et dialkyl(C₁-C₆)arylalkyle en C₁-C₆, mono- et dialcoxy (C₁-C₆)arylalkyle en C₁-C₆, mono- et dialkyl(C₁-C₆)arylalcoxy en C₁-C₆, mono- et dialcoxy(C₁-C₆)arylalcoxy en C₁-C₆, alkyle en C₁-C₆, bromoalkyle en C₁-C₆, chloroalkyle en C₁-C₆, fluoroakyle en C₁-C₆, alcoxy en C₁-C₆, monoalcoxy(C₁-C₆)alkyle en C₁-C₄, bromo, chloro et fluoro et n est le nombre entier 0, 1, 2 ou 3;
(d) R₃ est choisi dans le groupe comprenant les groupes alkyle en C₁-C₄, phényle, phényle substitués par monoalkyle en C₁-C₄, phényle substitués par monoalcoxy en C₁-C₄, phénylalkyle en C₁-C₂, phénylalkyle en C₁-C₂ substitués par monoalkyle en C₁-C₄, phénylalkyle en C₁-C₂ substitués par monoalcoxy en C₁-C₄, monoalcoxy(C₁-C₄)alkyle en C₂-C₃ et haloalkyle en C₁-C₄, et p est le nombre entier 0, 1 ou 2, la somme de n et p étant égale à 0, 1, 2 ou 3 et dans laquelle lorsque p est égal à 2, n est égal à 0; et
(e) B et B' sont chacun choisis dans le groupe comprenant :
(i) les groupes aryle non substitués, mono-, di- et trisubstitués, phényle et naphtyle;
(ii) les groupes hétéroaromatiques non substitués, mono- et disubstitués pyridyle, furannyle, benzofurann-2-yle, benzofurann-3-yle, thiényle, benzothién-2-yle, benzothién-3-yle, dibenzofurannyle, dibenzothiényle, carbazolyle et fluorényle, chacun desdits substituants de ces groupes aryle et hétéroaromatiques dans (e)(i) et (ii) étant choisis dans le groupe comprenant les groupes hydroxy, aryle, monoalcoxy(C₁-C₆)aryle, dialcoxy(C₁-C₆)aryle, monoalkyl-(C₁-C₆)aryle, dialkyl(C₁-C₆)aryle, bromoaryle, chloroaryle, fluoroaryle, cycloalkylaryle en C₃-C₇, cycloalkyle en C₃-C₇, cycloalkyloxy en C₃-C₇, cycloalkyloxy(C₃-C₇)alkyle en C₁-C₆, cycloalkyloxy(C₃-C₇)alcoxy en C₁-C₆, arylalkyle en C₁-C₆, arylalcoxy en C₁-C₆, aryloxy, aryloxyalkyle en C₁-C₆, aryloxyalcoxy en C₁-C₆, mono- et dialkyl(C₁-C₆)arylalkyle en C₁-C₆, mono- et dialcoxy(C₁-C₆)arylalkyle en C₁-C₆, mono- et dialkyl(C₁-C₆)arylalcoxy en C₁-C₆, mono- et dialcoxy(C₁-C₆)arylalcoxy en C₁-C₆, amino, monoalkylamino en C₁-C₆, dialkylamino en C₁-C₆, diarylamino, N-alkyl(C₁-C₆)pipérazino, N-arylpipérazino, aziridino, indolino, pipéridino, arylpipéridino, morpholino, thiomorpholino, tétrahydroquinoléino, tétrahydroisoquinoléino, pyrryle, alkyle en C₁-C₆, bromoalkyle en C₁-C₆, chloroalkyle en C₁-C₆, fluoroalkyle en C₁-C₆, alcoxy en C₁-C₆, monoalcoxy(C₁-C₆)alkyle en C₁-C₄, acryloxy, méthacryloxy, bromo, chloro et fluoro;
(iii) les groupes représentés par les formules graphiques suivantes : 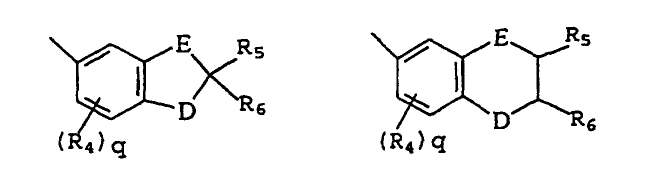 dans lesquelles E est du carbone ou de l'oxygène et D est de l'oxygène ou de l'azote substitué, pour autant que lorsque D est de l'azote substitué, E soit du carbone, lesdits substituants de l'azote étant choisis dans le groupe comprenant l'hydrogène et les groupes alkyle en C₁-C₆ et acyle en C₂-C₆; chaque R₄ est un groupe alkyle en C₁-C₆, alcoxy en C₁-C₆, hydroxy, bromo, chloro ou fluoro; R₅ et R₆ sont chacun de l'hydrogène ou un groupe alkyle en C₁-C₆, et q est le nombre entier 0, 1 ou 2;
(iv) les groupes alkyle en C₁-C₆, bromoalkyle en C₁-C₆, chloroalkyle en C₁-C₆, fluoroalkyle en C₁-C₆, alcoxy(C₁-C₆)alkyle en C₁-C₄; et
(v) le groupe représenté par la formule graphique suivante : 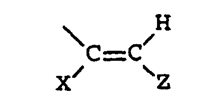 dans laquelle X est de l'hydrogène ou un groupe alkyle en C₁-C₄ et Z est choisi parmi les membres non substitués, mono- et disubstitués du groupe comprenant les groupes naphtyle, phényle, furannyle et thiényle, chacun des disubstituants de ces groupes étant un groupe alkyle en C₁-C₄, alcoxy en C₁-C₄, bromo, chloro ou fluoro.

2. Naphtopyranne suivant la revendication 1 dans lequel :
(a) chaque R₁ est un groupe alkyle en C₁-C₄, alcoxy en C₁-C₄, chloro ou fluoro et m est le nombre entier 0, 1 ou 2;
(b) Ar est un groupe phényle ou thiényle;
(c) chaque R₂ est choisi dans le groupe comprenant les groupes aryle, aryloxy, arylalkyle en C₁-C₃, alkyle en C₁-C₃, chloroalkyle en C₁-C₃, fluoroalkyle en C₁-C₃, alcoxy en C₁-C₃, monoalcoxy(C₁-C₃)alkyle en C₁-C₃, fluoro et chloro, et n est le nombre entier 0, 1 ou 2;
(d) R₃ est un groupe alkyle en C₁-C₃, et p est le nombre entier 0 ou 1, dans lequel lorsque p est égal à 1, n est égal à 0; et
(e) B et B' sont chacun choisis dans le groupe comprenant :
(i) les groupes phényle, phényle monosubstitués et phényle disubstitués;
(ii) les groupes hétéroaromatiques non substitués, monosubstitués et disubstitués furannyle, benzofurann-2-yle, thiényle, benzothién-2-yle, dibenzofurann-2-yle et dibenzothién-2-yle, chacun desdits substituants de ces groupes phényle et hétéroaromatiques dans (e)(i) et (ii) étant choisis dans le groupe comprenant les groupes hydroxy, aryle, aryloxy, arylalkyle en C₁-C₃, amino, monoalkylamino en C₁-C₃, dialkylamino en C₁-C₃, N-alkyl(C₁-C₃)pipérazino, indolino, pipéridino, morpholino, pyrryle, alkyle en C₁-C₃, chloroalkyle en C₁-C₃, fluoroalkyle en C₁-C₃, alcoxy en C₁-C₃, monoalcoxy(C₁-C₃)alkyle en C₁-C₃, fluoro et chloro;
(iii) les groupes représentés par les formules graphiques suivantes : 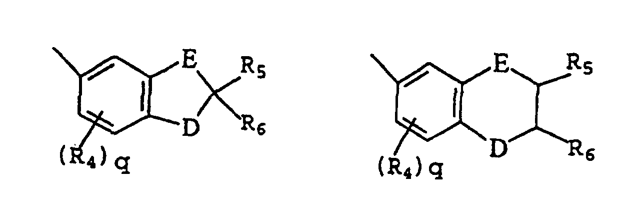 dans lesquelles E est du carbone et D est de l'oxygène, R₄ est un groupe alkyle en C₁-C₃ ou alcoxy en C₁-C₃, R₅ et R₆ sont chacun de l'hydrogène ou un groupe alkyle en C₁-C₄; et q est le nombre entier 0 ou 1;
(iv) un groupe alkyle en C₁-C₄; et
(v) le groupe représenté par la formule graphique suivante : 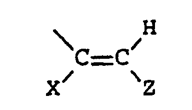 dans laquelle X est de l'hydrogène ou du méthyle et Z est un groupe phényle ou phényle monosubstitué, ledit substituant de phényle étant choisi dans le groupe comprenant les groupes alkyte en C₁-C₃, alcoxy en C₁-C₃ et fluoro.

3. Naphtopyranne suivant la revendication 2, dans lequel :
(a) R₁ est un groupe alkyle en C₁-C₃ ou alcoxy en C₁-C₃ et m est le nombre entier 0 ou 1;
(b) Ar est un groupe phényle ou thiényle;
(c) R₂ est un groupe alkyle en C₁-C₃, alcoxy en C₁-C₃, aryle, fluoro ou chloro; et n est le nombre entier 0 ou 1;
(d) R₃ est un groupe alkyle en C₁-C₃, et p est le nombre entier 0 ou 1, dans lequel lorsque p est égal à 1, n est égal à 0; et
(e) B et B' sont chacun choisis dans le groupe comprenant :
(i) les groupes phényle et phényle monosubstitués et disubstitués;
(ii) les groupes hétéroaromatiques non substitués, mono- et disubstitués furannyle, furann-2-yle, thiényle et benzothién-2-yle, chacun desdits substituants de ces groupes phényle et hétéroaromatiques dans (e)(i) et (ii) étant choisis dans le groupe comprenant les groupes hydroxy, alkyle en C₁-C₃, alcoxy en C₁-C₃, aryle, indolino, fluoro et chloro; et
(iii) le groupe représenté par la formule graphique suivante : 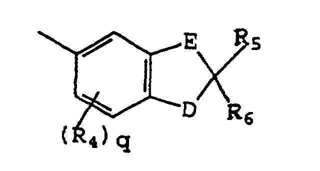 dans laquelle E est du carbone et D est de l'oxygène, R₄ est un groupe alkyle en C₁-C₃ ou alcoxy en C₁-C₃, R₅ et R₆ sont chacun de l'hydrogène ou un groupe alkyle en C₁-C₃; et q est le nombre entier 0 ou 1.

4. Composé de naphtopyranne choisi dans le groupe comprenant :
(a) le 2,2-diphényl-5-(2-méthoxycarbonylphényl)-2H-naphto[1,2-b]-pyranne; et
(b) le 2,2-diphényl-5-(thién-2-yl)-2H-naphto[1,2-b]pyranne.

5. Article photochromique comprenant une matière hôte organique polymérique et une quantité photochromique du composé de naphtopyranne de l'une quelconque des revendications 1 à 4.

6. Article photochromique comprenant, en combinaison, une matière hôte organique polymérique transparente solide et une quantité photochromique de chacun (a) d'au moins un composé de naphtopyranne de l'une quelconque des revendications 1 à 4 et (b) d'au moins un autre composé photochromique organique ayant un maximum d'absorption activé dans la gamme entre 400 et 700 nanomètres.

7. Article photochromique suivant la revendication 6, dans lequel le composé photochromique organique (b) est choisi dans le groupe comprenant d'autres naphtopyrannes, les chromènes, les oxazines, les dithizonates métalliques, les fulgides et les fulgimides.

8. Article photochromique suivant l'une quelconque des revendications 5 à 7, dans lequel la quantité totale de composé photochromique présent est de 0,05 à 1,0 mg par centimètre carré de surface de matière hôte organique à laquelle la ou les substances photochromiques sont incorporées ou appliquées.

9. Article photochromique suivant l'une quelconque des revendications 5 à 8, dans lequel la matière hôte est un polymérisat d'un monomère de résine organique optique.

10. Article photochromique suivant la revendication 9, dans lequel l'indice de réfraction du polymérisat est de 1,48 à 1,75.

11. Article photochromique suivant la revendication 10, dans lequel l'indice de réfraction du polymérisat est de 1,495 à 1,466.

12. Article photochromique suivant l'une quelconque des revendications 5 à 8, dans lequel la matière hôte organique polymérique est choisie dans le groupe comprenant les poly(méthacrylates d'alkyle en C₁-C₁₂), les poly(diméthacrylates d'oxyalkylène), les poly(méthacrylates phénoliques alcoxylés), l'acétate de cellulose, le triacétate de cellulose, l'acétopropionate de cellulose, l'acétobutyrate de cellulose, le poly(acétate de vinyle), le poly(alcool vinylique), le poly(chlorure de vinyle), le poly(chlorure de vinylidène), les polycarbonates thermoplastiques, les polyesters, les polyuréthannes, le poly(éthylène téréphtalate), le polystyrène, le poly(alpha-méthylstyrène), le copoly(styrène-méthacrylate de méthyle), le copoly(styrène-acrylonitrile), le polyvinylbutyral et les polymères des membres du groupe comprenant les monomères de bis(allyl carbonate), les monomères d'acrylate polyfonctionnels, les monomères de méthacrylate polyfonctionnels, les monomères de diéthylène glycol diméthacrylate, les monomères de diisopropényl benzène, les monomères de diméthacrylate de bisphénol A éthoxylé, les monomères d'éthylène glycol bisméthacrylate, les monomères de poly(éthylène glycol) bisméthacrylate, les monomères de bisméthacrylate de phénol éthoxylé, les monomères d'acrylate d'alcool polyatomique alcoxylé, les monomères de styrène, les monomères d'uréthanne acrylate, les monomères d'acrylate de glycidyle, les monomères de méthacrylate de glycidyle et les monomères de diallylidène pentaérythritol.

13. Artide photochromique suivant la revendication 12, dans lequel la matière hôte organique polymérique est un polymère transparent solide choisi dans le groupe comprenant le poly(méthacrylate de méthyle), le poly(éthylène glycol bisméthacrylate), le poly(diméthacrylate de bisphénol A éthoxylé), les polycarbonates thermoplastiques, le poly(acétate de vinyle), le polyvinylbutyral, les polyuréthannes et les polymères des membres du groupe comprenant les monomères de diéthylène glycol bis(allyl carbonate), les monomères de diéthylène glycol diméthacrylate, les monomères de bisméthacrylate de phénol éthoxylé, les monomères de diisopropényl benzène et les monomères de triméthylol propane triacrylate éthoxylé.

14. Article photochromique suivant l'une quelconque des revendications 5 à 13, dans lequel la matière hôte organique polymérique transparente précitée est un élément optique.

15. Article photochromique suivant la revendication 14, dans lequel ledit élément optique est une lentille.
